# EUROPEAN PATENT SPECIFICATION

(11) **EP 3 688 469 B1**
(45) Date of publication and mention of the grant of the patent: **21.08.2024**
(21) Application number: 18782137.6
(22) Date of filing: 25.09.2018
(51) Int. Cl.: G01N 33/68

(54) **PREDICTIVE BIOMARKER RATIO OF AKI**
PRÄDIKTIVE BIOMARKER-RATIO VON AKI
RAPPORT DE BIOMARQUEUR PRÉDICTIF D'AKI

(30) Priority: 25.09.2017 GB 201715511
(43) Date of publication of application: 05.08.2020
(73) Proprietor: Belfast Health And Social Care Trust, Belfast BT9 7AB (GB)
(72) Inventor: MCBRIDE, William Thomas, Belfast Antrim BT8 8BH (GB)
(74) Representative: Murgitroyd & Company
(86) International application number: PCT/GB2018/052728
(87) International publication number: WO 2019/058146

(56) References cited:
- WO-A1-2009/130505
- WO-A1-2013/186564
- WO-A2-2010/068686
- US-A1- 2016 169 901
- US-A1- 2016 169 901
- STANILOV NOYKO ET AL: "The prognostic value of preoperative serum levels of IL-12p40 and IL-23 for survival of patients with colorectal cancer", APMIS, vol. 122, no. 12, 7 June 2014 (2014-06-07), DK, pages 1223 - 1229, XP055870112, ISSN: 0903-4641, DOI: 10.1111/apm.12288
- BIBIANA BIELEKOVA ET AL: "Cerebrospinal Fluid IL-12p40, CXCL13 and IL-8 as a Combinatorial Biomarker of Active Intrathecal Inflammation", PLOS ONE, vol. 7, no. 11, 30 November 2012 (2012-11-30), pages e48370, XP055218549, DOI: 10.1371/journal.pone.0048370
- HO JULIE ET AL: "Urinary, Plasma, and Serum Biomarkers' Utility for Predicting Acute Kidney Injury Associated With Cardiac Surgery in Adults: A Meta-analysis", AMERICAN JOURNAL OF KIDNEY DISEASES, vol. 66, no. 6, 2015, pages 993 - 1005, XP029306588, ISSN: 0272-6386, DOI: 10.1053/J.AJKD.2015.06.018
- STANILOV NOYKO ET AL: "The prognostic value of preoperative serum levels of IL-12p40 and IL-23 for survival of patients with colorectal cancer", APMIS, vol. 122, no. 12, 7 June 2014 (2014-06-07), DK, pages 1223 - 1229, XP055870112, ISSN: 0903-4641, DOI: 10.1111/apm.12288
- BIBIANA BIELEKOVA ET AL: "Cerebrospinal Fluid IL-12p40, CXCL13 and IL-8 as a Combinatorial Biomarker of Active Intrathecal Inflammation", PLOS ONE, vol. 7, no. 11, 30 November 2012 (2012-11-30), pages e48370, XP055218549, DOI: 10.1371/journal.pone.0048370
- HO JULIE ET AL: "Urinary, Plasma, and Serum Biomarkers' Utility for Predicting Acute Kidney Injury Associated With Cardiac Surgery in Adults: A Meta-analysis", AMERICAN JOURNAL OF KIDNEY DISEASES, vol. 66, no. 6, 2015, pages 993 - 1005, XP029306588, ISSN: 0272-6386, DOI: 10.1053/J.AJKD.2015.06.018
- JUNG NAM AN ET AL: "Circulating tumour necrosis factor receptors 1 and 2 predict contrast-induced nephropathy and progressive renal dysfunction: A prospective cohort study : TNF receptors in CIN and renal deterioration", NEPHROLOGY, vol. 20, no. 8, 1 August 2015 (2015-08-01), AU, pages 552 - 559, XP055529748, ISSN: 1320-5358, DOI: 10.1111/nep.12448
- IGLESIAS JOSE ET AL: "Elevated serum levels of the type I and type II receptors for tumor necrosis factor-[alpha] as predictive factors for ARF in patients with septic s", AMERICAN JOURNAL OF KIDNEY DISEASES, vol. 41, no. 1, January 2003 (2003-01-01), pages 62 - 75, XP028914608, ISSN: 0272-6386, DOI: 10.1053/AJKD.2003.50024
- MCBRIDE W T ET AL: "Acute renal failure", SURGERY, MEDICINE PUBLISHING, ABINGTON. / ELSEVIER IMPRINT, GB, vol. 27, no. 11, 1 November 2009 (2009-11-01), pages 480 - 485, XP026735688, ISSN: 0263-9319, [retrieved on 20091031], DOI: 10.1016/J.MPSUR.2009.09.003

## Description

### Field of the Invention

The present invention relates to a method for determining predisposition of a subject to developing renal dysfunction, and to a kit for use in making such a determination.

### Background of the Invention

Apart from the direct adverse impact of physical trauma on the body (e.g. from lesions caused by surgery or car crashes, or from surgical procedures such as blood bypass through a heart-lung machine), subjects suffering physical trauma, which can include fractures, often develop acute renal dysfunction. Other causes of a similar acute renal dysfunction include prolonged hypotensive states (e.g. associated with mucosal gut ischaemia and endotoxin translocation from gut to circulation), sepsis and septic shock syndromes. Cardiac surgery can lead to acute renal dysfunction.

In a population of patients who have normal renal function, diagnosis of post-trauma, hypotensive-induced, sepsis-induced, and/or septic shock syndrome-induced renal dysfunction can be made on the basis of a fall in Glomerular Filtration Rate (GFR) as compared with the normal or baseline GFR; as measured, for example by the MDRD test (explained in more detail below). The limitation of a diagnosis or prognosis based on such measurements is that it usually takes several days for MDRD estimated GFR values to fall. When the GFR fall is detected, it is often too late to institute therapeutic measures to obviate further deterioration in renal function. The challenge facing for example the peri-operative physician or intensive care physician is to identify a biological marker of renal dysfunction 48 hours before such dysfunction materialises (i.e. while there is still a possibility of preventative measures being taken in the intensive care unit; such as running such patients on supra-normal blood pressures, an intervention which in its own right is not without risk in the postoperative context, unless justified by the presence of an even greater risk of impending renal failure such as could be identified by the test according to the present invention). Better still would be the ability to tell which individuals are most vulnerable to renal dysfunction should they experience any of a physically traumatic event, hypotensive event, sepsis or septic shock-syndrome. Such information would assist, for example, in assisting in the harm/benefit analysis required in order to determine if a subject should undergo surgery.

It is well documented that trauma to the body induces an acute (often transient) plasma pro-inflammatory response. It has been shown that the magnitude of this acute plasma pro-inflammatory response correlates with post-trauma renal dysfunction (Gormley et al., Anesthesiology, 2000, 93).

WO2009/130505 relates to a method for the diagnosis and/or prognosis of renal dysfunction induced by physical trauma, hypotension, sepsis and/or septic shock syndrome, wherein the method comprises the steps of:- a) determining the level of an anti-inflammatory cytokine present in a urine sample from a subject prior to physical trauma, prior to a hypotensive event, prior to sepsis, and/or prior to septic shock syndrome; b) determining the level of the anti-inflammatory cytokine present in a urine sample from the subject following physical trauma, following or during a hypotensive event, following or during sepsis, and/or following or during septic shock syndrome; c) calculating the difference between the level of the antiinflammatory cytokine determined in step a) from the level of the anti-inflammatory cytokine determined in step b); d) providing a diagnosis and/or prognosis on the basis of a comparison between the difference calculated in step c) and the difference calculated in step c) when steps a) and b) are practiced on a control group.

WO2013/186564 relates to a method for predicting the development of renal dysfunction in a subject following physical trauma, hypotension, sepsis and/or septic shock syndrome, wherein the method comprises the steps of:- a. determining the level of an anti-inflammatory cytokine present in a sample taken from the subject after physical trauma, after a hypotensive event, after sepsis, and/or after septic shock syndrome;
b. predicting the development in the subject of renal dysfunction on the basis of the level of an anti-inflammatory cytokine determined in step a).

Jung Nam An et al., Nephrology, vol 20, no. 8, 1 August 2015 pages 552 to 559 discloses elevated concentrations of circulating TNFRs were correlated with the occurrence of CIN and significantly associated with prolonged renal dysfunction regardless of the development of contrast-induced nephropathy (CIN) - an important cause of hospital-acquired acute kidney injury

Iglesias et al., Americal Journal of Kidney Diseases, vol. 41, no. 1, January 2003, pages 62 to 75 discloses elevated S-TNF-R levels may reflect a more intense inflammatory response.

### Summary of the invention

The inventors have found that specific anti-inflammatory markers are elevated in subjects prior to a physical trauma, and prior to a hypotensive event, prior to sepsis, and / or prior to septic shock syndrome allowing identification of those are predisposed to developing renal dysfunction prior to physical trauma. In particular, the invention is directed to a finding that anti-inflammatory markers are elevated in subjects pre-operatively in those subjects that have a greater than normal predisposition for developing renal dysfunction following physical trauma.

Accordingly, a first aspect of the present invention provides a method for determining predisposition of a subject to developing renal dysfunction induced by physical trauma, including fractures and cardiac surgery, hypotension, sepsis and / or septic shock syndrome, according to claim 1.

Suitably, TNFsr1 is Tumor Necrosis Factor Soluble Receptor 1, also known as p55.

Suitable TNFsr2 is Tumor Necrosis Factor Soluble Receptor 2, also known as p75.

Suitably, IL12p40 is a subunit of IL-12, wherein IL-12 is a 70-kDa heterodimeric cytokine composed of p35 and p40 subunits, wherein the p40 subunit may be detected on its own or as part of IL12p70 or as a subunit of IL-23 wherein Interleukin-23 (IL-23) is a heterodimeric cytokine composed of an IL-12p40 subunit (that is shared with IL-12) and the IL-23A (IL-23p19) subunit.

Suitably, the anti-inflammatory markers are elevated in subjects pre-operatively in those subjects that have a greater than normal predisposition for developing renal dysfunction following physical trauma. Suitably, in fracture subjects the fracture may considered a trauma, with the operation to repair or treat the fracture being an additional trauma. The fracture patients may be considered to be pre-conditioned by having already raised levels of cytokines. The method of the first aspect may be provided pre-operatively and post fracture. Suitably the control or normal values for fracture patients or patients who have undergone trauma prior to pre-operative trauma may be provided. Suitably such control values may be from a group of subjects that have had a fracture but not developed renal failure within, 24, 48, or 5 days post operatively.

Suitably, a control may be provided from healthy individuals or the individual themselves prior to trauma. Relative measurements or ratios could also be used.

Suitably a cardiac control may be different to a fracture control. Suitably for a cardiac control a measurement may be taken from the individual prior to any surgery. Measurements may then be taken post surgery. Control and samples for testing may be provided from plasma, serum and / or urine and could work with ratios of at least one, two or all biomarkers in all and between matrices.

A normal level would be determined from a control group on the same assay format of the method. The control group would not develop renal dysfunction.

Suitably a combination of TNFsr1, TNFsr2 and IL12p40 may be used in a method of the first aspect of the invention to determine a predisposition of a subject for developing renal dysfunction following physical trauma. Suitably a combination of TNFsr1, TNFsr2 and IL12p40 may be used in a method of the first aspect of the invention prior to a hypotensive event, prior to sepsis, and / or prior to septic shock syndrome.

Suitably a combination may be a combination of at least one of TNFsr1 or TNFsr2, and IL12p40.

Suitably determining levels of combinations of the biomarkers may allow greater sensitivity and specificity in diagnosing renal dysfunction. For example, ratios of the levels of biomarkers in biological samples may allow greater sensitivity and specificity.

In embodiments the sample can be from blood. Suitably the sample may be plasma or serum. In embodiments the sample can be urine.

In embodiments, when the level of TNFsr1, TNFsr2 or IL12p40 is lower than a normal level the subject has a lower than normal predisposition for developing renal dysfunction following physical trauma.

Suitably, the method may allow determination of a predisposition of renal dysfunction wherein the renal dysfunction is acute renal dysfunction. Suitably, the renal dysfunction may present 5-days or more following physical trauma, a hypotensive event, sepsis and / or septic shock. Suitably, renal dysfunction may be defined as a 25% or more decrease from normal glomerular filtration rate.

Suitably, a TNFsr1, TNFsr2 or IL12p40 level may be determined from a sample obtained from the subject within 24 hours prior to a physical trauma, a hypotensive event, sepsis, and / or septic shock syndrome.

The method of the present invention predicts the likelihood of the subject developing post-event (i.e. post-physical trauma, post-hypotensive event, or after the development of sepsis or septic shock syndrome or post-operative) renal dysfunction, should those events occur. The method is therefore a prognostic method. Subjects may be determined to have a greater than normal chance of developing renal dysfunction when they present with a TNFsr1, TNFsr2 or IL12p40 in a serum sample greater than a normal level prior to a trauma event.

A normal level is the level presented by a control group of individuals in their resting state, i.e. not following physical trauma, not following or during a hypotensive event, not following or during sepsis, and/or not following or during septic shock syndrome. Alternatively where appropriate, for example for fracture patients / subjects a control group may be post fracture, pre-operative group. The control group may comprise 30 individuals or more. The control group may comprise 300 individuals or more.

The normal level may be predetermined.

Renal dysfunction induced by physical trauma, hypotension, sepsis and/or septic shock syndrome may be further characterised by pro-inflammatory responses that are induced following these events. Such inflammatory responses commonly follow the pattern of a classical systemic inflammatory response (SIRS). Thus, the renal dysfunction to be prognosed according to the present invention may be those that are induced by an acute (possibly transient) urinary pro-inflammatory response (for example, elevated urinary levels of IL-18 and/or of neturophil gelatinase-associated lipocalin (NGAL)).

The renal dysfunction to be prognosed may be early renal dysfunction, late renal dysfunction, or general renal dysfunction. Early renal dysfunction occurs within two days of the event that induces the renal dysfunction. Late renal dysfunction occurs 5 days or later after such an event Determining whether or not an individual has renal dysfunction is a clinical question well within the abilities of the skilled person. However, in the interests of clarity, renal dysfunction is characterised by a reduction in the capacity to excrete metabolic products which accumulate systemically and are detectable clinicopathologically by renal function tests (in progressed states, renal dysfunction may be acute kidney failure, uremia or chronic renal damage). For example, renal dysfunction may be defined as a 25% or more decrease from normal glomerular filtration rate. Normal glomerular filtration rate is the pre-event rate. Glomerular filtration rate may be established in accordance with the MDRD study group formula. Such acute forms of renal dysfunction can be distinguished from autoimmune mediated chronic renal dysfunction, a condition that is clinically apparent over a prolonged period of time in parallel with the coexisting autoimmune condition (i.e. there is no requirement for a biological marker to predict the development of renal dysfunction occurring a few days later because the renal dysfunction is already well established).

The sample taken from the subject may be any sample capable of being analysed for the level of anti-inflammatory cytokine therein. For example, the sample may be a urine sample, a blood sample, for example a serum or a plasma sample. A serum sample is particularly preferred.

The samples analysed according to the first aspect of the invention may be obtained from the subject 48, 24 or 12 hours before the event (i.e. physical trauma, hypotensive event, onset of sepsis and/or onset of septic shock syndrome). The sample will optimally be obtained 24 hours before the event.

The predicting of the development in the subject of renal dysfunction in the present invention is based on the determination of higher levels of TNFsr1, TNFsr2 or IL12p40 present in a sample prior to a trauma event being indicative of a risk of renal dysfunction after an event.

A physical trauma is the impact on the body from external forces applied to the body, for example:- lesions caused by surgery or by blows or cuts to the body (such as might be experienced during a car crash), or; the impact on the blood as it interacts with the foreign surface of a heart-lung bypass machine. Renal dysfunction induced by physical trauma may be postoperative renal dysfunction. The post-operative renal dysfunction may be following cardiac, cardiovascular, cardiopulmonary or bone fracture surgery.

Whether or not an individual has hypotension is a clinical question and therefore well within the skill of an ordinary person in the art For the avoidance of doubt however hypotension in adults may be defined as a systolic blood pressure < 80 mmHg, or a mean arterial pressure (MAP) < 50 mmHg. The hypotension may be prolonged, for example for over 2 hours.

Whether or not an individual has sepsis is a clinical question and therefore well within the skill of an ordinary person in the art For the avoidance of doubt however, sepsis may be considered present if infection is highly suspected or proven and two or more of the following systemic inflammatory response syndrome (SIRS) criteria are met:
1. Heart rate > 90 beats per minute (tachycardia);
2. Body temperature < 36°C (97°F) or > 38 °C (100°F) (hypothermia or fever);
3. Respiratory rate > 20 breaths per minute or, on blood gas, a PₐCO₂ less than 32 mm Hg (4.3 kPa) (tachypnea or hypocapnia due to hyperventilation); and
4. White blood cell count < 4,000 cells/mm³ or > 12,000 cells/mm³ (< 4 x 10⁹ or > 12 x 10⁹ cells/L), or greater than 10% band forms.

Whether or not an individual has septic shock is a clinical question and therefore well within the abilities of an ordinary person skilled in the art For the avoidance of doubt however, septic shock may be defined by the presence of the following two criteria:
1. Evidence of infection, through a positive blood culture; and
2. Refractory hypotension - hypotension despite adequate fluid resuscitation and cardiac output In adults it is defined as a systolic blood pressure < 90 mmHg, or a MAP < 60 mmHg, before institution of required resuscitative inotropic support, or a reduction of 40 mmHg in the systolic blood pressure from baseline. In children it is BP < 2 SD of the normal blood pressure.

Whether or not an individual has SIRS is a clinical question and therefore well within the abilities of an ordinary person skilled in the art For the avoidance of doubt however SIRS may be diagnosed when two or more of the following are present:
1. Heart rate > 90 beats per minute
2. Body temperature < 36 or > 38°C
3. Tachypnea (high respiratory rate) > 20 breaths per minute or, on blood gas, a PₐCO₂ < 4.3 kPa (32 mm Hg)
4. White blood cell count < 4000 cells/mm³ or > 12000 cells/mm³ (< 4 x 10⁹ or > 12 x 10⁹ cells/L), or the presence of greater than 10% immature neutrophils.

The method may involve analysis of the levels of more than one type of anti-inflammatory cytokine. For example, the methods according to the present invention may further comprise the steps of :-
a) determining the level of one or more additional anti-inflammatory cytokine present in a sample taken from the subject prior to physical trauma, prior to a hypotensive event, prior to sepsis, and/or prior to septic shock syndrome, post fracture and pre-operative;
b) determining if the subject is predisposed to developing renal dysfunction following physical trauma, hypotension, sepsis and/or septic shock syndrome on the basis of the level of an anti-inflammatory cytokine determined in step a) and at least one of TNFsr2, TNFsr1 and IL12p40 according to the first aspect of the present invention.

In a second aspect of the present invention, there is provided use of a kit as claimed in claim 12.

The kit may further comprise one or more reagents for the detection of one or more anti -inflammatory mediator, and instructions for using the one or more reagents for detecting the one or more anti -inflammatory mediator.

The kit may further comprise instructions for using the detecting of the one or more anti-inflammatory cytokines in order to arrive at a prognosis for renal dysfunction. The instructions may be in accordance with the steps for prognosis of renal dysfunction provided in the first aspect of the present invention. The kit may further comprise instructions for using the detecting of the one or more anti-inflammatory mediator in order to arrive at a prognosis for renal dysfunction.

Also discussed herein is a method of treating renal dysfunction induced by physical trauma, hypotension, sepsis and/or septic shock syndrome, wherein the method includes the steps of: (i) prognosing renal dysfunction according to any of the methods of the first aspect of the present invention; and (ii) when the subject is identified to be at increased risk of developing renal dysfunction, applying therapeutic measures to treat or obviate the impending renal dysfunction. The advantage of such a method over current therapeutic interventions is that therapy may be administered at a stage when full renal failure may be prevented. The therapeutic measures applied in step (ii) may be: maintaining a supra-normal blood pressure; ensuring adequate tissue oxygen delivery; administration of steroids; renal replacement therapy; dialysis; or any combination thereof. A further advantage of this invention would be to allow intensive care managers to identify early in the intensive care stay of the patient those individuals who are likely to spend longer in intensive care than would otherwise be anticipated providing earlier planning for staff deployment

Discussed herein is a method for determining predisposition of a subject to developing renal dysfunction induced by physical trauma, including fractures and cardiac surgery, hypotension, sepsis and / or septic shock syndrome, wherein the method comprises the steps of:
determining the level of TNFsr1, TNFsr2 or IL12p40 present in a sample taken from the subject to following physical trauma, following a hypotensive event, following sepsis, and / or following to septic shock syndrome;
wherein when the level of TNFsr1, TNFsr2 or IL12p40 is higher than a normal level of TNFsr1, TNFsr2 or IL12p40 in a sample it is indicative the subject has a greater than normal predisposition for developing renal dysfunction following the physical trauma, hypotension, sepsis and / or septic shock syndrome event.

Suitably this method may be provided wherein the renal dysfunction induced by physical trauma is postoperative renal dysfunction, optionally wherein the post-operative renal dysfunction is following cardiac, cardiovascular, cardiopulmonary or bone fracture surgery.

A number of lists are provided above in which optional features for each of the aspects of the present invention are provided. Each member of the list is contemplated individually as a potential feature of the present invention.

The term "subject" may be any mammal, in particular a human, that is the recipient of a diagnosis. The subject may be provided with the method as part of routine screening or due to proposed symptoms of due to the presence of risk factors.

In the context of the present invention, a "control value" or "normal value" is the level of TNFsr1, TNFsr2 or IL12p40 by analysis of a sample isolated in a healthy individual or which is typical of a healthy individual or where appropriate in a post trauma (fracture), pre-operative group that does not develop renal dysfunction.

The "sample" may be any ex vivo biological sample from which the level of TNFsr1, TNFsr2 or IL12p40 can be determined.

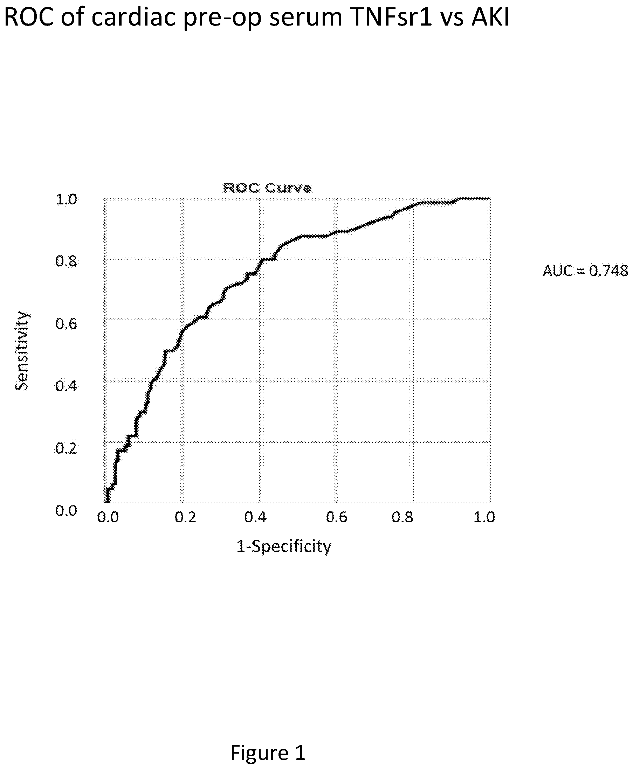

Determination of a level of TNFsr1, TNFsr2 or IL12p40 may be by any suitable method in the art For example, a suitable immunological method such as an ELISA-based assay or biochip assay. The sample may be provided with a probe capable of specifically binding to TNFsr1, TNFsr2 and / or IL12p40. Suitably alone or in combination with other detectors or labels the presence of a level of TNFsr1, TNFsr2 or IL12p40 can be determined. Suitably, the probe may be a solid state device comprising a substrate and a probe or multiple probes immobilized thereon which bind specifically to TNFsr1, TNFsr2 or IL12p40. Suitably the TNFsr1, TNFsr2 or IL12p40 can be detected by the specific binding of the probe(s). Suitably the probe may be an antibody, aptamer, phage, or oligonucleotide. Suitably, an antibody may be provided by Complementarity-Determining-Regions (CDRs) of molecules known in the art including monoclonal antibodies, polyclonal antibodies, Fab, Fab', and Fv fragments, single chain antibodies, or single chain variable fragments. Suitably an antibody may be conjugated to a label or detector, for example a radionuclide, fluorophore, dye, or enzyme. The level of TNFsr2, TNFsr1 or IL12p40 can be an expression level or quantitative concentration of the TNFsr2, TNFsr1 or IL12p40 or a relative level in comparison to a reference sample. The present invention will now be described, by way of example, with reference to the accompanying figures, in which:-
Figure 1 shows ROC of cardiac pre-op serum TNFsr1 vs AKI;

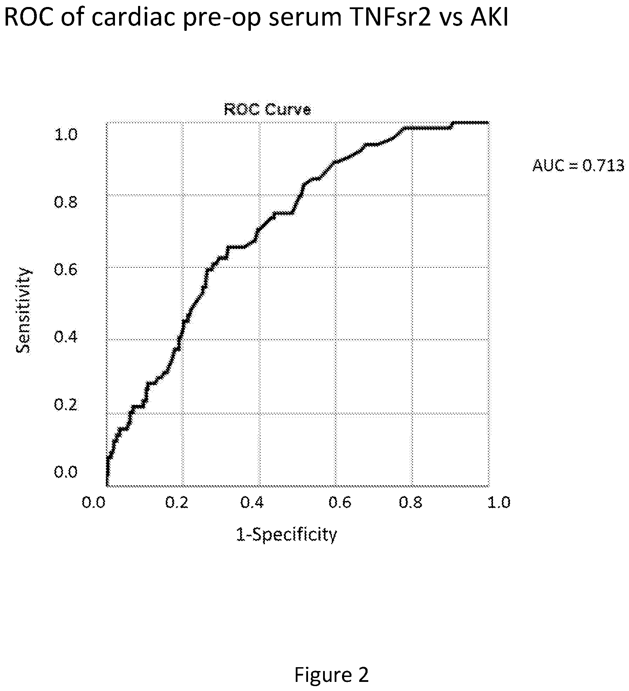

Figure 2 shows ROC of cardiac pre-op serum TNFsr2 vs AKI;

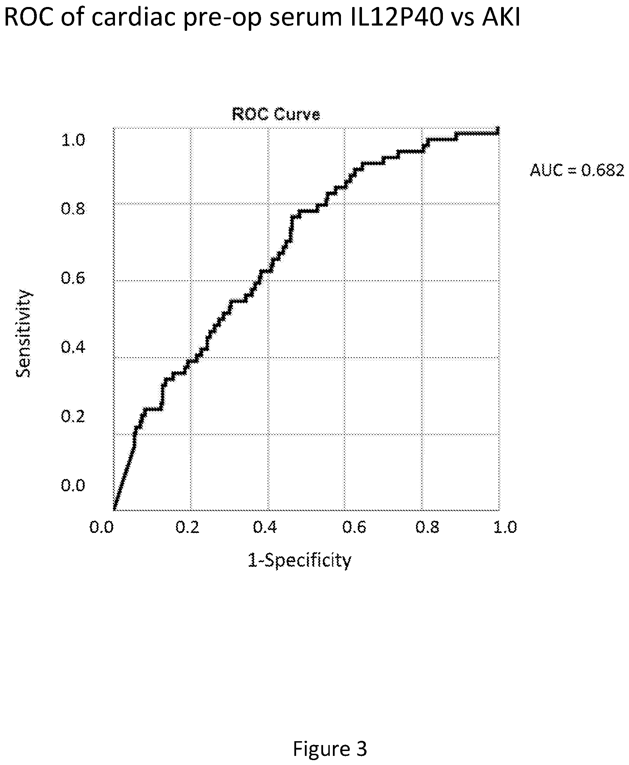

Figure 3 shows ROC of cardiac pre-op serum IL12p40;

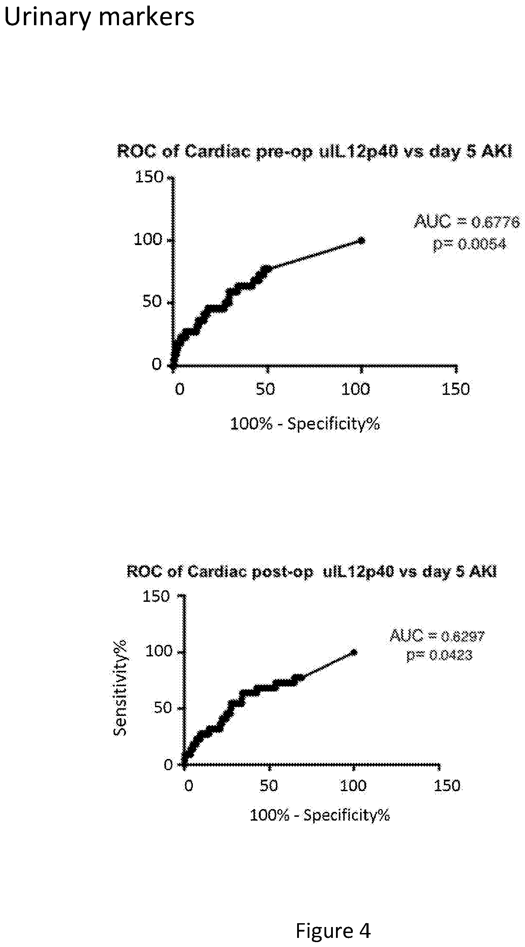

Figure 4 shows ROC of cardiac pre-op and post-op urinary IL12p40 vs 5 day AKI;

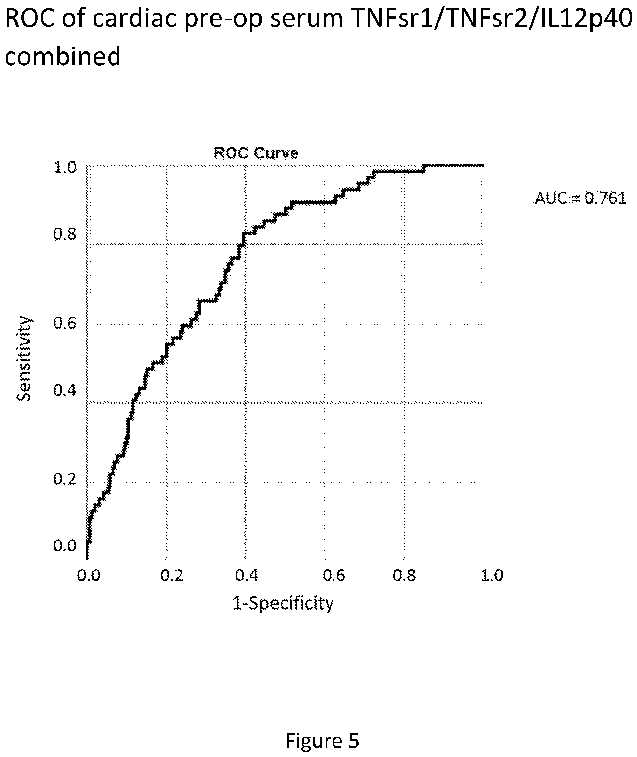

Figure 5 shows ROC of cardiac pre-op serum TNFsr1/TNFsr2/IL12p40 combined;

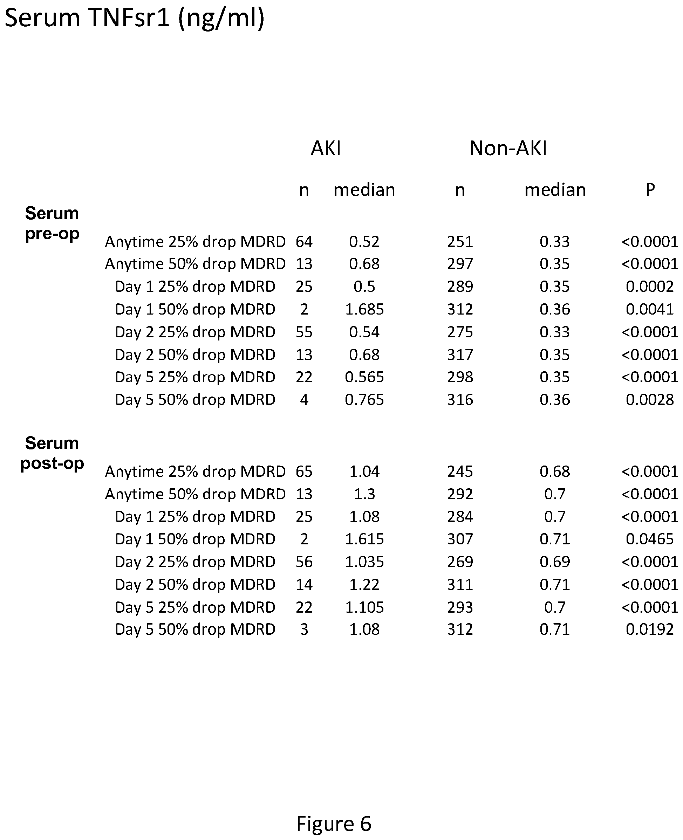

Figure 6 shows median values and probability for serum TNFsr1 between AKI and non-AKI Cardiac patients both pre and post-op;

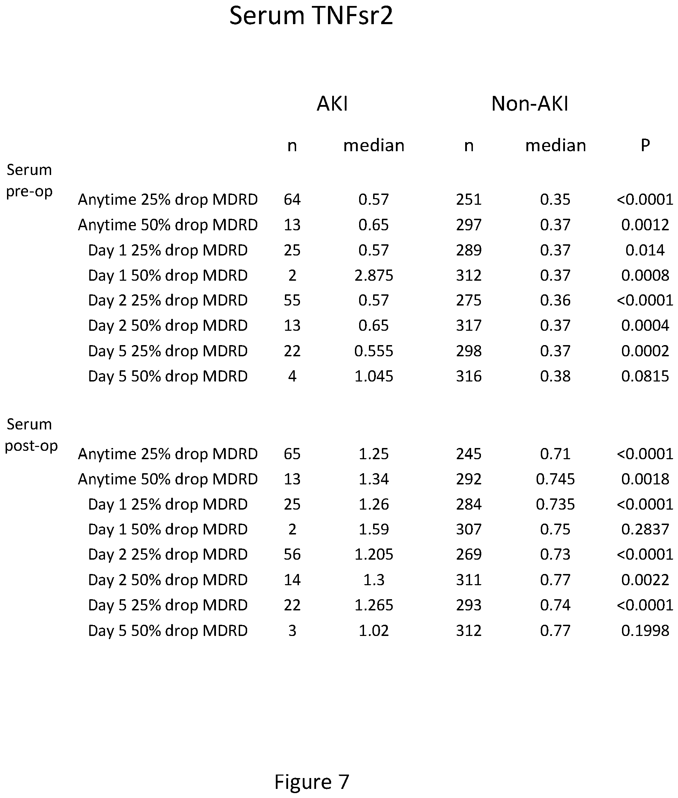

Figure 7 shows median values and probability for serum TNFsr2 between AKI and non-AKI Cardiac patients both pre and post-op;

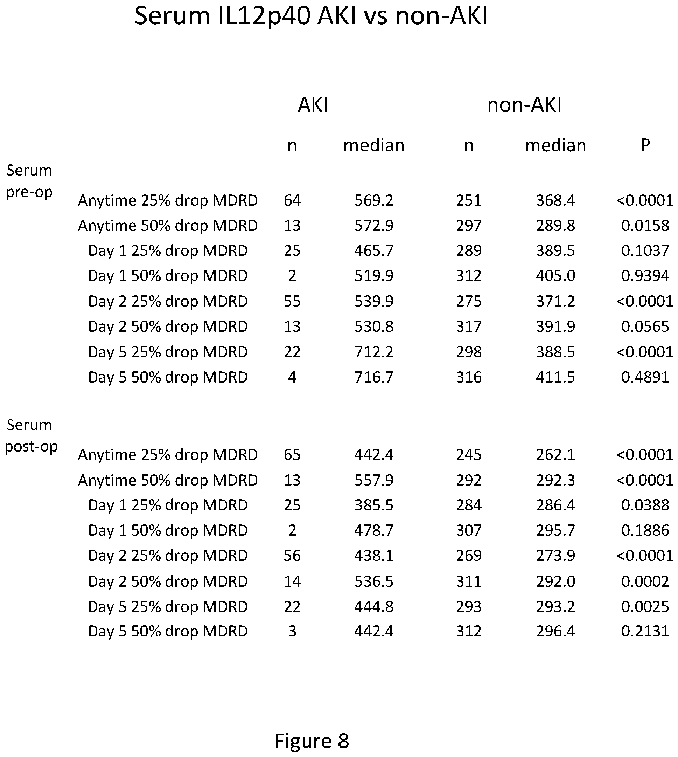

Figure 8 shows median values and probability for serum IL12p40 between AKI and non-AKI Cardiac patients both pre and post-op;

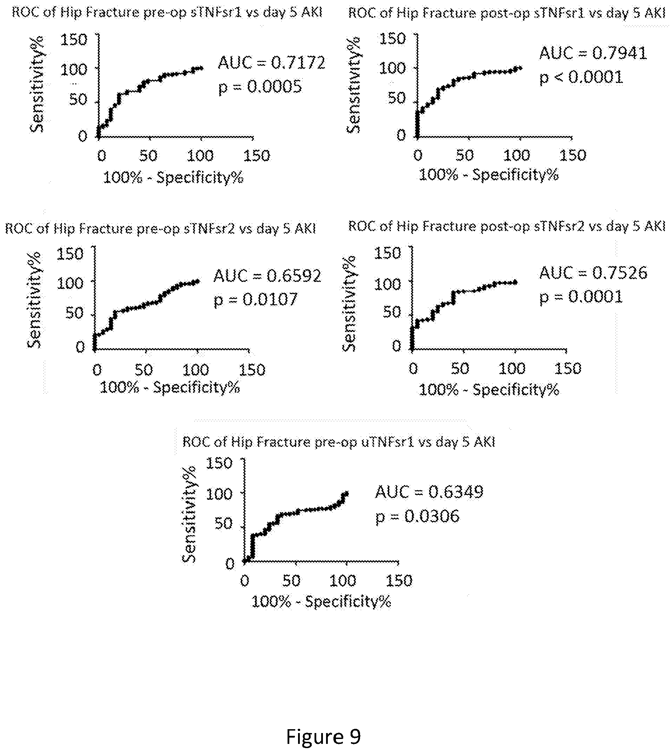

Figure 9 shows ROC for pre and post-op serum TNFsr1 and 2 in Hip fracture patients vs day 5 AKI; and

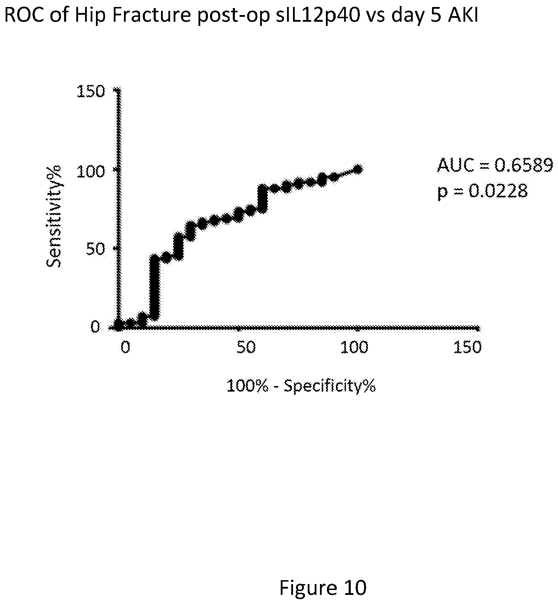

Figure 10 shows ROC for post-op serum IL12p40 in Hip fracture patients vs day 5 AKI.

### Experimental Methods

### Inclusion and exclusion criteria.

### Inclusion criteria for subjects:

i) Hip fracture patients
ii) Cardiac surgery elective patients
iii) Major trauma patients

### Exclusion criteria for subjects:

History of pre-existing renal dysfunction (eGFR < 30 mls/min/1.73m²), renal transplant or immunosuppressive or steroid therapy.

Nine hundred patients (450 for cardiac surgery and 450 for orthopaedic trauma) were recruited to the study (see power calculation below).

Cardiac surgery patients were consecutively scheduled for elective and emergency cardiac surgery within the Cardiac Surgical Unit of the Royal Victoria Hospital Belfast while the orthopaedic trauma patients were consecutively scheduled for open reduction internal fixation of their fracture within the fracture unit of the Belfast Trust. Exclusion criteria for all patients will be pre-operative or pre-trauma dialysis dependant renal failure or known significant renal disease prior to entrance into the study (known eGFR < 30).

There is evidence that 18% of fractured neck of femur patients have Rifle 1 criteria renal dysfunction at any time during their postoperative stay. It is not known how this number is distributed according to early and late presentation. Given the cardiac experience of half of all renal failure presentation being noted at day 5 it was assumed for this power calculation that 10% of patients will have day 5 late renal dysfunction.

On the basis of this, log transformation (base 10) of the median and interquartile ranges for Day 5 enabled two sample t-test methods to calculate the sample size based on these numbers. Using the interquartile range of the normal function group at day 5 a standard deviation (SD) of 0.45 was calculated based on the normal distribution. If we want to detect a difference of 0.2 at 80% power on the log10 scale between the groups (normal function/renal dysfunction) and assuming the SD is the same for both groups a sample size of 350 is needed, with 315 in the normal renal function group and 35 in the renal dysfunction group (i.e. on a ratio of 9:1 between groups). This calculation assumes that a two-tailed test at 5% significance will be applied and was carried out using Gpower 3.0.3.

If only fractured neck of femur patients are enrolled, then it is reasonable to expect at least 10% to fulfil Rifle criteria, in which case the number required is 350 patients. However, since some polytrauma patients were enrolled whose risk of renal dysfunction is even higher than the fracture neck of femur patients it is expected that to allow for wastage a minimum of 450 fractured neck of femur surgery and polytrauma patients will be required.

It is known that 5% of low risk cardiac surgery patients satisfy Rifle 1 criteria at day 5 and that 5% of all cardiac surgery patients have CRRT dependent renal dysfunction. The inventors have extrapolated that 10% of all cardiac surgery patients have day 5 renal dysfunction. On the basis of this and using the available pilot data on estimated glomerular filtration rate (eGFR) values at day 5, log transformation (base 10) of the median and interquartile ranges for Day 5 enabled two sample t-test methods to calculate the sample size based on these numbers. Using the interquartile range of the normal function group at day 5 a standard deviation (SD) of 0.45 was calculated based on the normal distribution. If the aim is to detect a difference of 0.2 at 80% power on the log10 scale between the groups (normal function/renal dysfunction) and assuming the SD is the same for both groups, a sample size of 350 consecutive low and high risk cardiac surgery patients is needed, with 315 in the normal renal function group and 35 in the renal dysfunction group (i.e. on a ratio of 9:1 between groups). This calculation assumes that a two tailed test at a 5% significance will be applied and was carried out using Gpower 3.0.3. To allow for wastage a minimum of 450 cardiac surgery patients is required.

Urinalysis (Sample A) (20ml) was performed on admission (for fracture patients) or for cardiac patients following catheterisation on induction of anaesthesia and (Sample B) (20ml) on day 1 post-operatively.

Blood Sample A (20ml) was performed on admission for fracture patients together with their routine pre-operative blood sample work-up such that participation in this study will not involve additional venepuncture. Blood sample A for cardiac patients will follow routine arterial line insertion pre-operatively. Day 1 post-operative Sample B (20ml) for fracture patients was taken at the time of routine analyses, hence not requiring further venepuncture. If, for a fracture patient, a blood sample is inadvertently missed during the time that routine bloods are being taken, was not be pursued to avoid additional discomfort of venepuncture beyond what is required for routine care, unless that patient, in intensive care or HDU had a routinely placed arterial catheter in situ. Unlike many fracture patients who do not have a routinely placed arterial line, all cardiac surgery patients have an arterial line inserted pre-operatively, remaining in situ for 48 hours such that obtaining the post-operative day 1 blood Sample B will be painless and the patient may well be unaware that this is happening.

Blood and urine was immediately centrifuged in the clinical area and the resulting supernatants stored in fridges.

### Cytokines analysis (ELISA)

Cytokines were measured by R&D systems Quantikine solid phase ELISA technique. This system consists of a conjugate, standard, assay diluent, calibrator diluent, wash buffer concentrate, colour reagent A, colour reagent B, and a stop solution. Reagents should be at the room temperature before beginning the assay. The microplate-consists of 96 wells. This microplate is coated with capture antibody. To each well assay diluent is added. Standards in duplicate and the samples are added to the plate and incubated for 2 h at room temperature. Any analyte present in the sample is bound by the capture antibody (immobilized antibody). After the incubation, the plate is aspirated and washed four times with the supplied wash buffer to washout any unbound materials. After washing, horseradish protease (HRP) labelled detection antibody (conjugate) is added to the plate and further incubated at room temperature. Once again, after the incubation the plate is aspirated and washed 4 times. Any unbound detection antibody is washed away. In the next step prepared substrate solution tetramethylbenzidine (TMB) is added to the wells and a blue colour develops in proportion to the amount of analyte present in the sample. After 20 minutes incubation the colour develops (blue) proportional to the cytokine concentration. For analysis colour development is stopped turning the colour in the wells to yellow. The absorbance of the colour at 450 nm is measured which is read in the microplate reader.

### Measurement of Renal dysfunction

In 1989 Kopple et al as part of the Modification of Diet in Renal Disease study group published their findings investigating the impact of nutritional status on chronic renal insufficiency in 95 patients. The effects on progression of renal disease of a control diet of only mild dietary protein restriction were compared with 3 study diets of varying degrees of protein restriction and reduced phosphorus intake. The authors found that malnutrition and lower energy intake characterised patients with the lower GFR levels. There were some gender differences with men demonstrating a correlation between GFR and arm muscle area and percentage standard body weight especially at the onset of the experimental diets. In women, GFR correlated with dietary energy intake [ Kopple et al., Kidney lnt Suppl 1989;27]. A logical development of this study from the MDRD group was the idea that analysis of patients' age weight, gender and ethnicity together with serum creatinine would allow GFR to be estimated. This assumption recognised that serum creatinine concentration alone does not adequately reflect renal function but should be considered along with the factors identified as influencing renal function in Kopple's study.

To develop the prediction equation 1628 patients were enrolled in the baseline period, of which 1070 were randomly selected as the training sample whereas the remaining 558 patients constituted the validation sample. The authors then used stepwise regression to the training sample to develop the equation which was then tested and compared with the Cockcroft and Gault formula and creatinine clearance measurements in the validation sample.

It was found that several measured variables were associated with a lower GFR. These included higher serum creatinine, higher serum urea and lower serum albumin levels concentrations. Independent variables associated with lower GFR included older age group, female gender and non-black ethnicity (P < 0.001 for all factors).

The multiple regression models explained 90.3% of the variance in the logarithm of GFR in the validation sample. Measured creatinine clearance overestimated GFR by 19%, and creatinine clearance predicted by the Cockcroft-Gault formula overestimated GFR by 16%. After adjustment for this overestimation, the percentage of variance of the logarithm of GFR predicted by measured creatinine clearance or the Cockcroft-Gault formula was 86.6% and 84.2%, respectively.

MDRD study group estimated GFR is calculated from the following formula:
X = 32788 x creatinine-1.154 x age-0.203 x constant where the constant is 1 for white males, 0.724 for females, and 1.21 for African Americans.

MDRD estimated GFR in the present study was calculated from the above formula. According to the above formula MDRD GFR was calculated at preop day 0 and at post operative days 1,2 and 5. For each separate post op day as well as all post op days together patients can be divided into normal and abnormal renal function groups where "normal" and "abnormal" were defined by those who sustained falls in MDRD GFR of less than or greater than 25% of baseline respectively.

In summary for post surgery cytokine values were compared between the normal and abnormal groups where normality and abnormality were defined according to the 4 definitions mentioned below.
Definition 1 : 'Abnormality' is defined as having day 1 MDRD >25% drop from baseline. (Early renal dysfunction).
Definition 2. 'Abnormality' is defined as having day 2 MDRD >25% drop from baseline. (Early renal dysfunction).
Definition 3. 'Abnormality' is defined as having day 5 MDRD >25% drop from baseline. (Late renal dysfunction).
Definition 4. 'Abnormality' is defined as having at least one MDRD value >25% drop from baseline during days 1, 2, and 5. (General renal dysfunction).

### Statistical methods

Quantitative variables were summarized as mean and standard deviation except for those with heavily skewed distributions for which median and interquartile range were used. Comparisons between groups were obtained using the independent samples z-test or Mann-Whitney U test for quantitative variables, and the chi-squared test or Fisher's exact test for categorical variables. The ability of cytokine measurements to distinguish patients with and without renal dysfunction in the preoperative period was examined using the receiver-operator characteristic (ROC) curve. The area under this curve provides a measure of discriminatory ability; a value of 1 indicates perfect discrimination while a value of 0.5 indicates no more discriminatory ability than would be expected by chance.

### Results

WO2013/186565 discloses a biomarker method of predicting renal dysfunction in an individual induced by physical trauma, hypotension, sepsis and/or septic shock syndrome which involves measuring the level of an anti-inflammatory cytokine IL-1ra prior to the physical insult and wherein when a high level of the cytokine IL-1ra was determined compared to that of a normal level it is indicative of a lower risk of developing renal dysfunction.

The current study has produced the surprising finding that a higher than normal serum level of the anti-inflammatory cytokines sTNFr1, sTNFr2 and IL12p40 pre cardiac surgery is indicative of a higher risk of developing renal dysfunction. This held true for patients who developed AKI day 2 and day 5 post surgery and for patients who developed AKI at any time post surgery (Table 1). The markers also showed utility post-surgery. Further, it appears that fracture subject that have already been compromised by the fracture itself leading to raised cytokine levels may be effectively 'pre-conditioned' and such subjects may be less likely to develop renal failure than for example a cardiac subject that has not had an initial cytokine spike.

**Table 1 AUC values and probabilities for serum TNFsr1, TNFsr2 and IL12p40 measured pre and post-op vs Day 2, Day 5 and all AKI.**

| | **AKI on Day 2** | | | | **AKI on Day 5** | | | | **AKI at any day** | | | |
|---|---|---|---|---|---|---|---|---|---|---|---|---|
| | **Pre** | | **Post** | | **Pre** | | **Post** | | **Pre** | | **Post** | |
| | **AUC** | **P** | **AUC** | **P** | **AUC** | **P** | **AUC** | **P** | **AUC** | **P** | **AUC** | **P** |
| **STNFRI (transf ormed)** | 0.762 | <0. 001 | 0.758 | <0. 001 | 0.772 | <0. 001 | 0.802 | <0. 001 | 0.748 | <0. 001 | 0.774 | <0.001 |
| **STNFRI I (transf ormed)** | 0.710 | <0. 001 | 0.746 | <0. 001 | 0.751 | 0.0 01 | 0.762 | <0. 001 | 0.713 | <0. 001 | 0.762 | <0.001 |
| **SIL-12P40 (transf ormed)** | 0.661 | 0.0 01 | 0.693 | <0. 001 | 0.746 | 0.0 04 | 0.679 | 0.0 01 | 0.682 | <0. 001 | 0.714 | <0.001 |
| **No. AKI** | 54 | | | | 21 | | | | 63 | | | |
| **No. Non-AKI** | | 266 | | | 289 | | | | 253 | | | |
| | | | | | | | | | | | | |

The combination of all three biomarkers pre-op in serum for cardiac patients gave an AUC of 0.761. Two marker combinations pre-op in serum for cardiac patients gave the following AUCs; TNFsr1 and TNFsr2 - 0.715, TNFsr1 and IL12p40 - 0.756 and TNFsr2 and IL12p40 - 0.739.

## Claims

1. A method for determining predisposition of a subject to developing renal dysfunction induced by physical trauma, hypotension, sepsis and / or septic shock syndrome, wherein the method comprises the steps of: determining the level of IL12p40 present in a sample taken from the subject prior to physical trauma, prior to a hypotensive event, prior to sepsis, and / or prior to septic shock syndrome; wherein when the level of IL12p40 in the sample is higher than a normal level (control level) of IL12p40 in a sample it is indicative the subject has a greater than normal predisposition for developing renal dysfunction following physical trauma, hypotension, sepsis and / or septic shock syndrome.

2. The method as claimed in claim 1 wherein a combination of each of TNFsrl, TNFsr2 and IL12p40 are used to determine a predisposition of a subject for developing renal dysfunction following physical trauma, hypotension, sepsis and / or septic shock syndrome.

3. The method as claimed in claim 1 wherein a combination of at least one of TNFsrl or TNFsr2, and IL12p40 is used for determining predisposition of a subject to developing renal dysfunction induced by physical trauma, hypotension, sepsis and / or septic shock syndrome.

4. The method as claimed in any one of claims 1 to 3 wherein the sample is obtained from blood, optionally wherein the sample is plasma or serum.

5. The method as claimed in any one of claims 1 to 3 wherein the sample is urine.

6. The method as claimed in any one of claims 1 to 5 wherein when the level of IL12p40 is lower than a normal level the subject has a lower than normal predisposition for developing renal dysfunction following physical trauma, hypotension, sepsis and / or septic shock syndrome.

7. The method as claimed in any preceding claim wherein the predisposition of renal dysfunction is a predisposition to acute renal dysfunction.

8. The method as claimed in any preceding claim wherein the renal dysfunction may be present 5-days or more following physical trauma, a hypotensive event, sepsis and / or septic shock.

9. The method as claimed in any preceding claim wherein renal dysfunction is defined as a 25% or more decrease from normal glomerular filtration rate.

10. The method as claimed in any preceding claim wherein the level is determined from a sample obtained from the subject within 24 hours prior to a physical trauma, hypotensive event, sepsis, and / or septic shock syndrome.

11. The method a claimed in any preceding claim wherein the renal dysfunction induced by physical trauma is postoperative renal dysfunction, optionally wherein the postoperative renal dysfunction is following cardiac, cardiovascular, cardiopulmonary or bone fracture surgery.

12. Use of a kit in the methods of the preceding claims, wherein the kit comprises:- one or more reagents to detect - instructions for determining whether the is higher than a normal level as observed in a subject prior to a physical trauma event, or prior to trauma selected from hypotensive event, sepsis, and / or septic shock syndrome.

13. Use of a kit as claimed in claim 12 wherein the kit further comprises one or more reagents for the detection of one or more anti-inflammatory mediator selected from TNFsr1 and TNFsr2.

## Patentansprüche

1. Ein Verfahren zur Bestimmung der Prädisposition eines Individuums für die Entstehung einer durch körperliches Trauma, Hypotonie, Sepsis und/oder septisches Schocksyndrom induzierten Nierenfunktionsstörung, wobei das Verfahren die folgenden Schritte beinhaltet: Bestimmen des Spiegels von IL12p40, der in einer dem Individuum vor dem körperlichen Trauma, vor einem Hypotonieereignis, vor der Sepsis und/oder vor dem septischen Schocksyndrom entnommenen Probe vorhanden ist; wobei, wenn der Spiegel von IL12p40 in der Probe höher ist als ein normaler Spiegel (Kontrollspiegel) von IL12p40 in einer Probe, dies anzeigt, dass das Individuum eine höhere als die normale Prädisposition für die Entstehung einer Nierenfunktionsstörung nach körperlichem Trauma, Hypotonie, Sepsis und/oder septischem Schocksyndrom aufweist.

2. Verfahren gemäß Anspruch 1, wobei eine Kombination von jedem von TNFsr1, TNFsr2 und IL12p40 verwendet wird, um eine Prädisposition eines Individuums für die Entstehung einer Nierenfunktionsstörung nach körperlichem Trauma, Hypotonie, Sepsis und/oder septischem Schocksyndrom zu bestimmen.

3. Verfahren gemäß Anspruch 1, wobei eine Kombination von mindestens einem von TNFsr1 oder TNFsr2 und IL12p40 verwendet wird, um die Prädisposition eines Individuums für die Entstehung einer durch körperliches Trauma, Hypotonie, Sepsis und/oder septisches Schocksyndrom induzierten Nierenfunktionsstörung zu bestimmen.

4. Verfahren gemäß einem der Ansprüche 1 bis 3, wobei die Probe aus Blut erhalten wird, wobei die Probe optional Plasma oder Serum ist.

5. Verfahren gemäß einem der Ansprüche 1 bis 3, wobei die Probe Urin ist.

6. Verfahren gemäß einem der Ansprüche 1 bis 5, wobei, wenn der Spiegel von IL12p40 niedriger ist als ein normaler Spiegel, das Individuum eine niedrigere als die normale Prädisposition für die Entstehung einer Nierenfunktionsstörung nach körperlichem Trauma, Hypotonie, Sepsis und/oder septischem Schocksyndrom aufweist.

7. Verfahren gemäß einem der vorhergehenden Ansprüche, wobei die Prädisposition für Nierenfunktionsstörung eine Prädisposition für akute Nierenfunktionsstörung ist.

8. Verfahren gemäß einem der vorhergehenden Ansprüche, wobei die Nierenfunktionsstörung 5 oder mehr Tage nach körperlichem Trauma, einem Hypotonieereignis, Sepsis und/oder septischem Schocksyndrom vorliegen kann.

9. Verfahren gemäß einem der vorhergehenden Ansprüche, wobei Nierenfunktionsstörung als eine 25%ige oder höhere Abnahme gegenüber der normalen glomerulären Filtrationsrate definiert ist.

10. Verfahren gemäß einem der vorhergehenden Ansprüche, wobei der IL12p40-Spiegel aus einer Probe bestimmt wird, die von dem Individuum innerhalb von 24 Stunden vor einem körperlichen Trauma, einem Hypotonieereignis, einer Sepsis und/oder einem septischen Schocksyndrom erhalten wurde.

11. Verfahren gemäß einem der vorhergehenden Ansprüche, wobei die durch körperliches Trauma induzierte Nierenfunktionsstörung eine postoperative Nierenfunktionsstörung ist, wobei die postoperative Nierenfunktionsstörung optional nach einer Herz-, Herz-Kreislauf-, Herz-Lungen- oder Knochenfraktur-Operation eingetreten ist.

12. Verwendung eines Kits in den Verfahren gemäß den vorhergehenden Ansprüchen, wobei der Kit Folgendes beinhaltet: - ein oder mehrere Reagenzien zum Nachweis von IL12p40 - Anweisungen zur Bestimmung, ob der IL12p40-Spiegel höher ist als ein normaler Spiegel, wie er bei einem Individuum vor einem körperlichen Traumaereignis oder vor einem Trauma, ausgewählt aus Hypertonieereignis, Sepsis und/oder septischem Schocksyndrom, beobachtet wurde.

13. Verwendung eines Kits gemäß Anspruch 12, wobei der Kit ferner ein oder mehrere Reagenzien zum Nachweis eines oder mehrerer antientzündlicher Mediatoren, ausgewählt aus TNFsr1 und TNFsr2, beinhaltet.

## Revendications

1. Une méthode pour déterminer une prédisposition d'un sujet à développer un dysfonctionnement rénal induit par un traumatisme physique, une hypotension, une septicémie et/ou un syndrome de choc septique, la méthode comprenant les étapes consistant à : déterminer le niveau de IL12p40 présent dans un échantillon prélevé sur le sujet préalablement à un traumatisme physique, préalablement à un événement hypotensif, préalablement à une septicémie, et/ou préalablement à un syndrome de choc septique ; dans laquelle lorsque le niveau de IL12p40 dans l'échantillon est plus élevé qu'un niveau normal (niveau de contrôle) de IL12p40 dans un échantillon, cela indique que le sujet a une prédisposition supérieure à la normale à développer un dysfonctionnement rénal suite à un traumatisme physique, à une hypotension, à une septicémie et/ou à un syndrome de choc septique.

2. La méthode telle que revendiquée dans la revendication 1 dans laquelle une combinaison de chaque élément parmi TNFsrl, TNFsr2 et IL12p40 est utilisée pour déterminer une prédisposition d'un sujet à développer un dysfonctionnement rénal suite à un traumatisme physique, une hypotension, une septicémie et/ou un syndrome de choc septique.

3. La méthode telle que revendiquée dans la revendication 1 dans laquelle une combinaison d'au moins un élément parmi TNFsrl ou TNFsr2, et IL12p40 est utilisée pour déterminer une prédisposition d'un sujet à développer un dysfonctionnement rénal induit par un traumatisme physique, une hypotension, une septicémie et/ou un syndrome de choc septique.

4. La méthode telle que revendiquée dans n'importe laquelle des revendications 1 à 3 dans laquelle l'échantillon est obtenu à partir de sang, facultativement dans laquelle l'échantillon est du plasma ou du sérum.

5. La méthode telle que revendiquée dans n'importe laquelle des revendications 1 à 3 dans laquelle l'échantillon est de l'urine.

6. La méthode telle que revendiquée dans n'importe laquelle des revendications 1 à 5 dans laquelle lorsque le niveau de IL12p40 est inférieur à un niveau normal, le sujet a une prédisposition inférieure à la normale à développer un dysfonctionnement rénal suite à un traumatisme physique, une hypotension, une septicémie et/ou un syndrome de choc septique.

7. La méthode telle que revendiquée dans n'importe quelle revendication précédente dans laquelle la prédisposition à un dysfonctionnement rénal est une prédisposition à un dysfonctionnement rénal aigu.

8. La méthode telle que revendiquée dans n'importe quelle revendication précédente dans laquelle le dysfonctionnement rénal peut se présenter 5 jours ou plus à la suite d'un traumatisme physique, d'un événement hypotensif, d'une septicémie et/ou d'un choc septique.

9. La méthode telle que revendiquée dans n'importe quelle revendication précédente dans laquelle le dysfonctionnement rénal est défini comme une diminution de 25 % ou plus par rapport à un débit de filtration glomérulaire normal.

10. Une méthode telle que revendiquée dans n'importe quelle revendication précédente dans laquelle le niveau de IL12p40 est déterminé à partir d'un échantillon obtenu du sujet dans les 24 heures préalablement à un traumatisme physique, un événement hypotensif, une septicémie, et/ou un syndrome de choc septique.

11. La méthode telle que revendiquée dans n'importe quelle revendication précédente dans laquelle le dysfonctionnement rénal induit par un traumatisme physique est un dysfonctionnement rénal post-opératoire, facultativement dans laquelle le dysfonctionnement rénal post-opératoire fait suite à une chirurgie cardiaque, cardiovasculaire, cardiopulmonaire ou de fracture osseuse.

12. Utilisation d'un kit dans les méthodes des revendications précédentes, le kit comprenant : - un ou plusieurs réactifs afin de détecter le IL12p40 - des instructions pour déterminer si le IL12p40 est plus élevé qu'un niveau normal tel qu'observé chez un sujet préalablement à un événement de traumatisme physique, ou préalablement à un traumatisme sélectionné parmi un événement hypotensif, une septicémie, et/ou un syndrome de choc septique.

13. Utilisation d'un kit telle que revendiquée dans la revendication 12, le kit comprenant en outre un ou plusieurs réactifs pour la détection d'un médiateur anti-inflammatoire ou plus sélectionné parmi TNFsr1 et TNFsr2.
